# EUROPEAN PATENT SPECIFICATION

(11) **EP 3 253 200 B1**
(45) Date of publication and mention of the grant of the patent: **13.11.2019**
(21) Application number: 16719479.4
(22) Date of filing: 03.03.2016
(51) Int. Cl.: A01N 1/02, A01N 43/90, A61K 47/61, C08B 37/00, A01P 3/00

(54) **SYNTHETIC ANTIMYCOTIC MOLECULE**
SYNTHETISCHES ANTIMYKOTISCHES MOLEKÜL
MOLÉCULE ANTIMYCOTIQUE SYNTHÉTIQUE

(30) Priority: 04.03.2015 IT UB20150108
(43) Date of publication of application: 13.12.2017
(73) Proprietor: Alchilife S.r.l., 35020 Ponte San Nicolò PD (IT)
(72) Inventor: BECCARO, Mauro, 35010 Cadoneghe (PD) (IT); BETTINI, Enrico, 30032 Fiesso D'Artico (VE) (IT); SIGNORI, Paolo, 37100 Verona (VR) (IT)
(74) Representative: Ponchiroli, Simone
(86) International application number: PCT/IB2016/051205
(87) International publication number: WO 2016/139619

(56) References cited:
- WO-A1-85/02975

## Description

This invention relates to a synthetic antimycotic molecule and a method for synthesising an antimycotic molecule.

In particular, this invention relates to a synthetic antimycotic molecule that is biocompatible relative to human organs and tissues and that, at least at temperatures between 0°C and 40°C, is highly soluble in water.

This invention was conceived following requirements that arose in the sector of ophthalmic surgery, in particular with reference to the storage and decontamination of explanted corneas intended for transplantation. However, subsequently, given the results achieved, it was understood how this invention could also have many other applications in the medical and surgical sector, as described below.

At present, in the ophthalmic sector in general, and in that of cornea transplants in particular, it is established practice that many compositions are used, both in the form of medical devices and in the form of medicines.

In particular, like all surgeries, even those in the ophthalmic sector expose the patient to the risk of infections which could arise during post-operation recovery. Therefore, to reduce said risk, it is routine procedure to use various types of antibiotic substances not just during the surgery and/or the subsequent convalescence, but also during the step of storing the explanted corneas intended for transplantation. In fact, as for any other tissue or organ, it is essential to guarantee that the biological material to be implanted in the patient (the cornea) is free of contamination. In fact, otherwise, the patient would be exposed to a high risk of subsequent onset of infections. Basically, according to some statistics, cases of post-operative endophthalmitis appear to have been increasing in recent years.

According to the prior art in the cornea transplants sector, to remove any contamination from the explanted cornea, a known technique is to store it at least temporarily in a bath containing in addition to many other ingredients, a cocktail of antibiotics such that they provide cover for a wide spectrum. However, at present there are obvious limits concerning the antibiotics used in such cocktails, since whilst there are suitable antibiotics present for gram positive and gram negative bacteria, there are no antimycotic substances present. The reason for this is that, at least in the temperature range between 0°C and 40°C (corneas are usually stored either at 4°C or at 37°C depending on the storage technique) none of the known antimycotic molecules is soluble enough in water (the solubility is usually such that it does not even guarantee reaching the concentration necessary for having a minimal fungicidal action). This makes it difficult, if not practically impossible to use them in liquids for storing or processing corneas, which are all water-based (it should be noticed that whilst the definition storage liquid refers to a liquid in which the cornea can be stored for relatively lengthy periods - several days - also thanks to the presence of nutrients, the definition processing liquid refers to a liquid with which the cornea can be put into contact only for a limited period of time, either because it contains no nutrients or because in the long-term it is potentially toxic for the cornea. Examples of processing liquids are decontaminating liquids, washing liquids and deturgescence liquids).

It should also be noticed that in an attempt to get around the problem of the insolubility of the prior art antimycotic agents, the use is known of amphotericin B bound in micellar form to deoxycholate. In fact, the micelle of these two substances has a certain "solubility" in water that allows its use in solution. However, what is obtained is not a solution but a heterogeneous mixture. Consequently, due to its high molecular weight, as time passes the amphotericin B-deoxycholate micelle tends to hyperconcentrate towards the bottom of the container. However, said hyperconcentration corresponds to an increase in toxicity which, if used in a liquid for storing and/or processing corneas, would risk compromising the cornea. In addition, some studies have cast doubt on the biocompatibility of deoxycholate.

Obviously, similar problems in the prevention and/or treatment of mycotic infections are encountered in all medical applications in which it would be appropriate to have available an antimycotic agent that is soluble in water.

It should be remembered that EP 1000541, EP 517972 and US 5104787 describe serum-free medical solutions for storing explanted corneas, which are composed of a nutrient aqueous solution, with the addition of many components including glycosaminoglycans and antibiotics. In the only quantitative example provided in EP 1000541, the antibiotics used are gentamicin and streptomycin, whilst in the only one provided in both EP 517972 and in US 5104787, the only antibiotic used is gentamicin. All three documents also mention many other antimycotic agents that would be usable, but no further related explanations are supplied, or even information about the composition that could be obtained using them.

Document WO 2006/076663, concerning pelvic problems, describes compositions usable for the treatment, amelioration or prevention of one or more pelvic symptoms such as pain, urgent urination, frequent urination or incontinence. Such compositions are in the form of a mixture and in general comprise an anionic polysaccharide, an anaesthetic and a buffer for controlling the pH. Amongst the many anionic polysaccharides indicated as preferred in WO 2006/076663, the only one used in the specific examples supplied in it is heparin. WO 2006/076663 also describes many other optional components of the composition, including an antibacterial agent or an antimycotic agent (the latter selected in the group comprising amphotericin B, itraconazole, ketoconazole, fluconazole, miconazole and flucytosine). However, no specific example of use is provided.

Therefore, none of the four patent documents referred to describes any new antimycotic molecule, let alone an antimycotic molecule that is free of the above-mentioned disadvantages.

Finally WO 85/02975 describes a composition comprising chondroitin sulphate and amphotericine B, in which the two molecules are simply mixed; no chemical link exists between the molecule of chondroitin sulphate and the molecule of amphotericine B.

In this context the technical purpose which forms the basis of this invention is to provide a synthetic antimycotic molecule which overcome the above-mentioned disadvantages.

In particular, the technical purpose of this invention is to provide a synthetic antimycotic molecule that is biocompatible with human organs and tissues (at least for the contact times involved in the above-mentioned applications) and that has a high level of solubility in water, at least such that it ensures a concentration which guarantees fungicidal action at biocompatible temperatures (approximately between 0°C and 40°C).

Furthermore, the technical purpose of this invention is to develop a water-based composition for storing and/or processing corneas and/or other explanted tissues or organs which also has suitable antimycotic properties. The technical purpose specified and the aims indicated are substantially achieved by a synthetic antimycotic molecule as described in the appended claims.

Further features and the advantages of this invention are more apparent in the detailed description below of several preferred, non-limiting embodiments of a synthetic antimycotic molecule and of a method for synthesising an antimycotic molecule.

The synthetic antimycotic molecule according to this invention is constituted of a chain of n basic units, where each basic unit of the chain has the following structure: 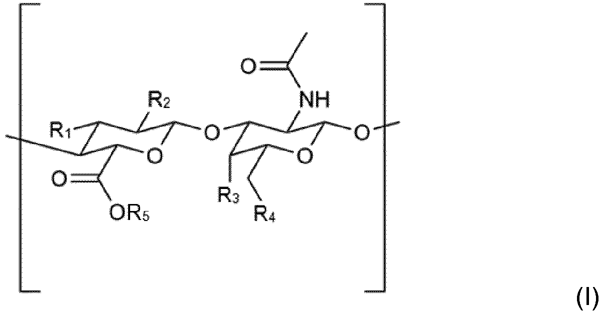 where:
- for each basic unit of the chain, one of either R₃ or R₄ is a sulphate group with the structure: 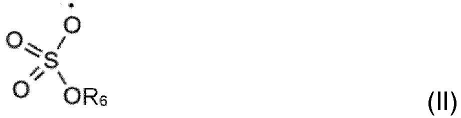 (in the graphical representation of the group structure, the dot represents the point of the bond with the rest of the respective basic unit) with R₆ representing either a hydrogen atom, or one of either a metallic atom or a group NH₄, bound to the corresponding oxygen atom O with a monovalent ionic bond. In other words, when R₆ represents a metallic atom or a group NH₄, the chain is salified compared with the case in which R₆ is constituted of a hydrogen atom H;
- for a plurality of basic units of the chain, at least one of R₁, R₂, or the one of either R₃ and R₄ which is not the sulphate group referred to in the previous point, is a group with antimycotic properties in turn having the structure: 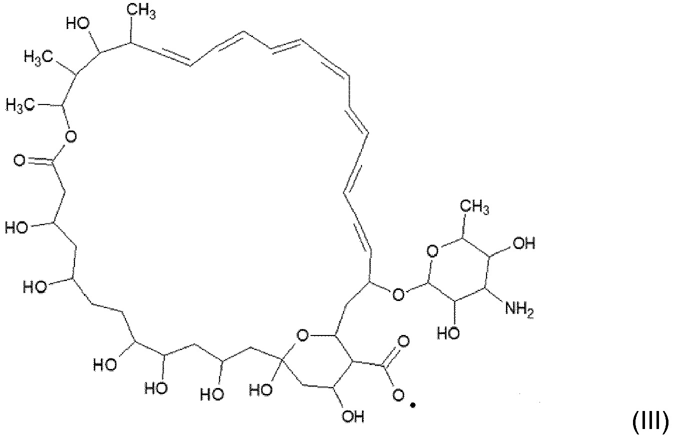 (again in this case, in the graphical representation of the structure of the group, the dot represents the point of the bond with the rest of the respective basic unit);
- for each basic unit of the chain, R₅ represents either a hydrogen atom, or one of either a metallic atom or a group NH₄, bound to the corresponding oxygen atom O with a monovalent ionic bond. In other words, when R₅ represents a metallic atom or a group NH₄, the chain is salified compared with the case in which R₅ is constituted of a hydrogen atom H;
- at least most of the remaining R₁, R₂, R₃ and R₄ of the entire chain are each constituted of a -OH group.

As regards the number n of basic units present in the molecule according to this invention, it may vary according to requirements. In particular, since it is a chain, said number n will always be greater than or equal to 2, whilst, as is also explained in more detail below, at the moment there are no known reasons for setting a maximum value for the number n. However, since there are currently no prior art methods for synthesising the molecule according to this invention other than that described below, based on which the skeleton of the antimycotic molecule of this invention corresponds to the skeleton of a chondroitin sulphate molecule, the value n may be assumed to be equal to any number of repeating units of a chondroitin sulphate molecule. Consequently, the maximum value for the number n will be equal to the maximum number of repeating units that can naturally be found in a chondroitin sulphate molecule. At present, there are no chondroitin sulphate molecules available with more than approximately 482 repeating units. In the preferred applications of this invention, the number n of basic units is advantageously greater than or equal to 2, preferably greater than or equal to 24 and even more preferably greater than or equal to 48, whilst it is advantageously less than or equal to 482, preferably less than or equal to 361, and even more preferably less than or equal to 150.

However, in the preferred embodiments all of the remaining R₁, R₂, R₃ and R₄, which are not a sulphate group or a group with antimycotic properties, are each constituted of a -OH group.

Moreover, preferably, each basic unit of the chain comprises a maximum of only one of said groups with antimycotic properties, even if in less preferred embodiments it is possible that they may also comprise more than one of them.

Moreover, advantageously, not all of the basic units comprise one or more groups with antimycotic properties. On the contrary, in accordance with the preferred embodiments of the molecule, the number of basic units of the chain that have at least one group with antimycotic properties is within the range between 1% and 50% of the total number of basic units.

Depending on requirements, the sulphate group may occupy the same position in all of the basic units, or not. However, in the preferred embodiment, it is always in the same position and corresponds to the group R₄ of the formula (I).

As regards R₅ and R₆ in the preferred embodiments they are each advantageously constituted of a sodium atom Na, of a potassium atom K or of an ammonium cation NH₄.

In addition to the molecule itself, this invention also relates to the compositions comprising a water-based mixture, in which the antimycotic molecule described above is also present.

In particular, in such compositions advantageously the antimycotic molecule is advantageously dissolved in the water (the water and antimycotic molecule therefore together form a solution).

Moreover, preferably, the mixture comprises the antimycotic molecule in a weight/volume percentage of between 1% and 30%, and even more preferably between 2% and 10%.

In the preferred embodiments of this invention, the mixture is in the form of hydrogel at least within a range of temperatures between 0°C and 40°C.

In particular, amongst all of the possible compositions within the scope of this invention, of particular importance are liquids for storing and/or processing explanted organs and/or tissues intended for transplantation which contain the molecule or the mixture described above.

Moreover, the scope of this invention also includes use of the antimycotic molecule or of a mixture which contains it, for decontaminating explanted organs or tissues intended for transplantation, as well as the corresponding treatment method.

As may be inferred from the above, in the context of this invention when reference is made to explanted organs or tissues intended for transplantation, what is meant is organs or tissues which are explanted from a first subject and intended for transplantation in a second subject who is different from the first subject.

Considering the antimycotic properties shown by the molecule according to this invention and the fact that it has proven to be biocompatible with human organs and tissues, this invention also relates in general to the uses of the antimycotic molecule described above or of a mixture that contains it as a medicine, as well as in particular the uses of it in the treatment of mycotic infections and in ophthalmic surgery.

Finally, as already indicated, it is also described a method for synthesising an antimycotic molecule, in particular of the type described above, in which the molecule is obtained by functionalisation of a molecule of chondroitin sulphate or of a salt of chondroitin sulphate or by means of an ester bond with one or more molecules of Amphotericin B, which, once bonded to the molecule of chondroitin sulphate constitute the above-mentioned antimycotic group. It should be noticed that what is described relative to the method shall also be understood to apply with reference to the molecule, and vice versa, if compatible.

In more detail, said method comprises first the operating steps of taking chondroitin sulphate or a salt of chondroitin sulphate, and taking some amphotericin B.

As regards the chondroitin sulphate, it is constituted of a chain of n basic units, each of which has the structure define by the formula (I) indicated above, where both R5 and R6 are either constituted of a hydrogen atom or are absent (therefore leaving the related oxygen atom in negative ion form), whilst as regards the amphotericin B, each molecule has a carboxyl-terminus with the structure -COOH.

As is known, chondroitin sulphate is a molecule that is naturally available in various forms, and can only be obtained by extraction from animal sources, not synthesised in a laboratory. Consequently, it is known that the length of the chondroitin sulphate molecule (that is to say, the number n of basic units - repeating units - that it has) may vary greatly, from a minimum value in which the chain has a number n of basic units equal to 2, to an undefinable maximum number (molecules with n greater than approximately 480 are not currently available). However, the length of the chondroitin sulphate chain is not decisive for the purposes of this invention, which can be implemented using chains of any length. However, in some applications, use is required of chondroitin sulphate with a chain length of between 2 and 150 basic units, and even more preferably between 6 and 130 basic units.

Moreover, as already indicated, the number n of repeating units (basic units) of the starting chondroitin sulphate molecule, corresponds to the number n of basic units of the synthetic antimycotic molecule with the formula (I) obtainable at the end of the synthesising method.

Preferably, the chondroitin sulphate or the salt of chondroitin sulphate have a molecular weight of between approximately 10 kDa and 200 kDa, and even more preferably of between approximately 20 kDa and 150 kDa. Since a basic unit of chondroitin sulphate has a molecular weight of approximately 415 Da, the above also means that, according to this invention, the number n of basic units of chondroitin sulphate (which corresponds to the number n of basic units of the chain of the antimycotic molecule defined by the formula (I) indicated above) is between 24 and 482, and even more preferably between 48 and 361.

Therefore, the method comprises functionalising the chondroitin sulphate or the salt of chondroitin sulphate, creating an ester bond between the carboxyl-terminus of the amphotericin B and a hydroxyl group of the chondroitin sulphate or of the salt of chondroitin sulphate.

In the preferred embodiments, the method also comprises a step of activation of the carboxyl-terminus of the amphotericin B in order to facilitate formation of the above-mentioned ester bond, increasing the chemical reactivity of the amphotericin B. Advantageously, the activation step may involve making the amphotericin B react with a coupling agent such as 1-isobutoxycarbonyl-2-isobutoxy-1,2-dihydroquinoline (IIDQ) or with an agent such as 1,1'-carbonyldiimidazole (CDI) which is able to convert a carboxylic acid into the corresponding imidazolide, the reactive form of an acylating agent.

Advantageously, in the case of use of CDI, first a first reaction of the free carboxylic group of the amphotericin B with the carbonyldiimidazole (CDI) is caused. The reaction occurs at temperatures of between 35°C and 40°C and lasts for 1-2 hours. That reaction is followed by functionalisation of the chondroitin sulphate, for example with a reaction caused at ambient temperature for 24 hours. In contrast, in the case of use of the IIDQ, initially there is mixing of the two components of the reaction (chondroitin sulphate and amphotericin B) in an aprotic solvent such as N-methlypyrrolidone (NMP) or dimethyl sulfoxide (DMSO), and then the addition of a stoichiometric quantity of IIDQ. The reaction occurs at a temperature of between 25°C and 40°C.

The preferred embodiment of this invention, in which chondroitin sulphate is used, since chondroitin sulphate is a very hydrophilic compound, whilst amphotericin B is hydrophobic, involves making one of the reagents compatible with the appropriate solvents for the other reagent. Advantageously, this result can be achieved by including a preliminary step in which the chondroitin sulphate is converted into an ammonium salt, such as the salt cetyltrimethylammonium (CTA) or, preferably, the salt of tetrabutylammonium (TBA). The ammonium salt may be formed by simply neutralising chondroitin sulphate with the hydroxide of the ammonium cation, or by ion exchange in sulfonic resins. It is a reaction that proceeds with 100% yield, and the resulting product is soluble in dimethyl sulfoxide (DMSO) or N-methlypyrrolidone (NMP) in which amphotericin B is also soluble.

The chondroitin sulphate substituted with ammonium and the "activated" amphotericin B compound (in the case of use of CDI) are then made to react in DMSO or NMP. For example, the reaction may occur in 24 hours at 40 °C. From a chemical viewpoint, the reaction consists of condensation of the carboxylic functionality of the amphotericin B (activated with CDI) with the hydroxyl group in the N-acetylgalactosamine units of the chondroitin sulphate. The ratio of units of N-acetylgalactosamine with which the amphotericin B is bonded to the total number of such units present in the chondroitin sulphate may advantageously be between 1% and 50%.

Once the molecule according to this invention has been obtained, it can be added to water, which can normally be double-distilled water, to a buffer saline solution, to a saline solution or to a culture medium, in a quantity such that its resulting concentration is between 1% and 30% w/v, preferably between 2% and 10% w/v, obtaining one of the above-mentioned compositions according to this invention.

By acting as described above, all of the concentrations of the antimycotic molecule in water between 1% and 30% w/v give uniform transparent solutions, often in the form of hydrogel.

### EXPERIMENTAL RESULTS

The following is a discussion of the experimental results obtained relative to an anti-fungal molecule synthesised according to this invention (hereinafter and in the accompanying plates called "Amphotericin B-Chondroitin"), whose stability and antimycotic activity were examined in comparison with those of both pure amphotericin (hereinafter and in the accompanying plates referred to as "Amphotericin B-insoluble"), and the amphoterocin+deoxycholate micellar mixture (hereinafter and in the accompanying plates referred to as "Amphotericin B-deoxycholate").

### Amphotericin B-Chondroitin Synthesis

In a 50 ml flask 220 mg (0.2 eq.) of amphotericin B were dissolved in NMP, and, upon completion of solubilisation, 74 mg (0.2 eq.) of IIDQ were added. The reaction mixture was then left being agitated for 20 minutes at ambient temperature.

Then 400 mg (1 eq.) of tetrabutylammonium salt of chondroitin sulphate (chondroitin TBA) with a molecular weight within the range 20-30 kDa were added, and the reaction left to run at ambient temperature over night.

The next morning, it was all transferred into a 500 ml beaker.

Then the following were added in sequence:
- 10 ml of water, very slowly and with agitation;
- 5 ml of saturated solution of NaCl, one drop at a time and with agitation;
- 50 ml of acetone, slowly and with agitation.

At that point, the precipitate was allowed to settle.

Then the supernatant was eliminated by carrying out 2 washes, each using 50 ml of cold acetone.

The residue was then washed with a cold mixture of Ethanol/water 80/20, until the NaCl was completely eliminated (test with AgNO₃ on supernatant). Four washes, each using 50 ml were required.

After completely eliminating the NaCl, washing with 100% ethanol was performed (2 washes, each using 50 ml).

Finally, washing with 100% acetone was performed (2 washes, each using 50 ml).

The operations of washing and elimination of the supernatant were carried out using a Gooch G4-type crucible (or a 0.22 µm or 0.45 µm filter capsule), taking care never to completely dry out the precipitate.

Alternatively, the precipitate plus the washing solution could have been centrifuged, with centrifuging not higher than 3000 rpm, and the supernatant eliminated by suction.

The mixture was then dialyzed against MilliQ water, using a 15 KDa cut-off membrane for 5 days, and the dialyzate was filtered with a 0.45 µm filter. The product obtained by lyophilisation was equal to 180 mg.

### Methods

The tests were carried out by adding the various substances to cornea storage liquids. In particular, the commercial liquids used were:
- EUSOL-C marketed by Al.Chi.Mi.A. S.r.l. of Ponte S. Nicolò (Padua province), Italy: a liquid for storing corneas at 4°C for periods of up to fourteen days; and
- OPTISOL-GS marketed by Bausch & Lomb Incorporated of Rochester, NY, USA: again a liquid for storing corneas at 4°C.

First, for each storage liquid (EUSOL-C and OPTISOL-GS) three different sterile solutions were prepared:
- solution 1: solution with 10 µg/ml of Amphotericin B-Chondroitin;
- solution 2: solution with 10 µg/ml of Amphotericin B-deoxycholate;
- solution 3: solution with 10 µg/ml of Amphotericin B-insoluble.

Two further solutions were also prepared as a control:
- solution 4 (first control): solution with 10 µg/ml of a mixture of amphotericin-deoxycholate in water;
- solution 5 (second control): solution with 10 µg/ml of pure amphotericin in DMSO.

All of the solutions were then stored in bottles at 4°C for fourteen days without agitation. On the fourteenth day after the final examination, the bottle was agitated and a further examination was carried out.

The antimycotic activity of each solution was tested using the diffusion on agar test. In particular, after 0, 1, 2, 7 and 9 days of storage at 4°C, each solution was picked up and placed on the agar plates seeded with Candida Albicans (CA). The diameter of the CA growth inhibition halo, induced by each solution, was measured on the plate after 24h of incubation at 31°C. For each time interval, 6 samples of each solution, corresponding to 3 pickups from two different bottles, were tested.

### Results

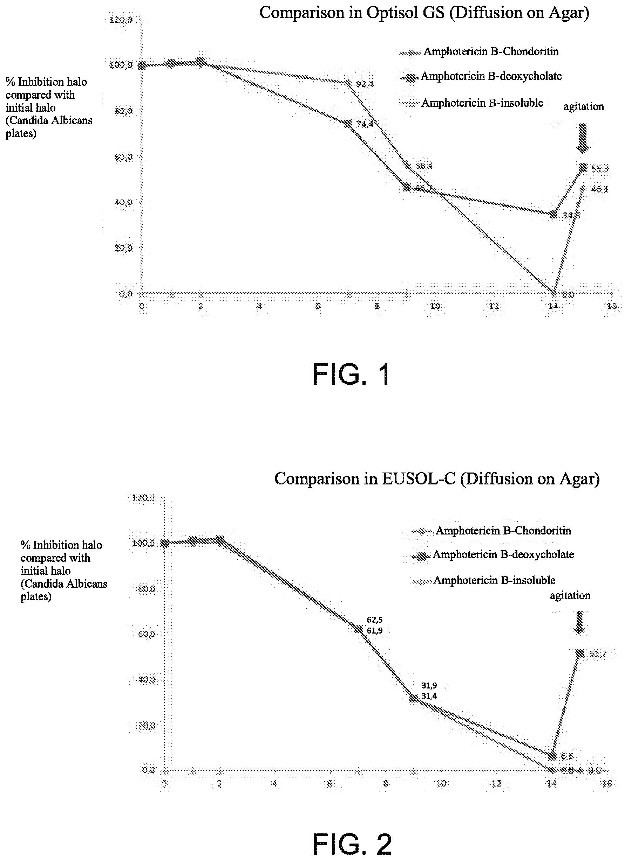

Figure 1 shows the results obtained for solutions 1 to 3 in OPTISOL-GS. In particular, the graph shows the trend of the inhibition halo generated by the solutions on a plate in agar. The y-axis values represent the percentage variation of the diameter of the halo compared with the initial value (day 0), whilst the x-axis values represent the time expressed in days.

Figure 2 is a graph similar to that of Figure 1, relating to the case of the solutions in EUSOL-C.

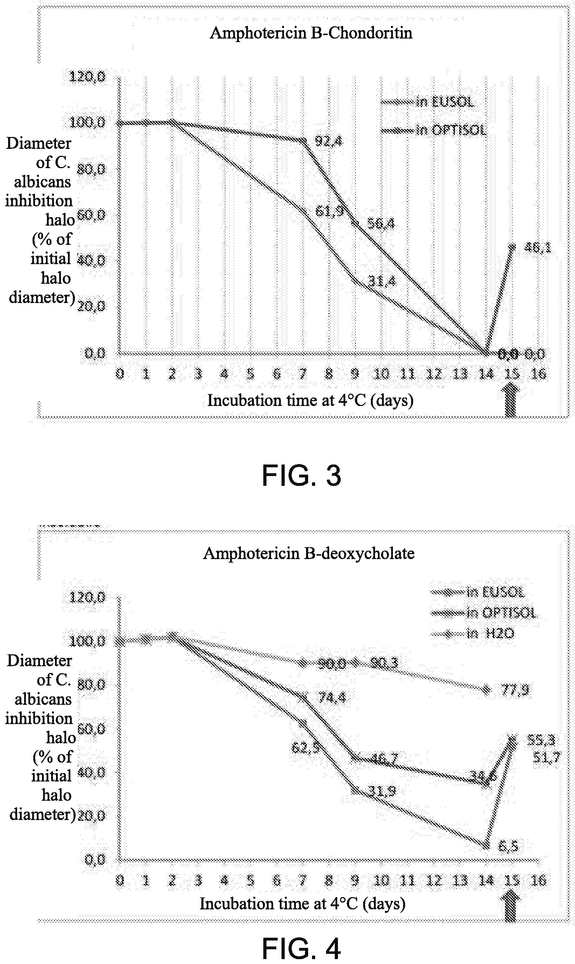

Figures 3 and 4 represent the same data, respectively for solution 1 and solution 2, of Figures 1 and 2, in a different format and with the addition in Figure 4 of the trend of the inhibition halos for the control solution 4. The arrow indicates the moment when the bottle was subjected to agitation.

As regards the behaviour of the OPTISOL-GS based solutions, as can be seen in Figure 1, for up to nine days of storage at 4°C the activity of solution 1 (with Amphotericin B-Chondroitin) was higher than the activity of solution 2 at each time analysed. After fourteen days of storage, solution 1 did not form any inhibition halo, whilst solution 2 formed a halo with a diameter equal to 35% of the diameter of the initial halos. After agitation, performed, as already indicated, only after fourteen days of storage, solution 1 and solution 2 formed halos with a diameter respectively equal to 46% and 55% of the initial diameter. The activity of solution 1 in OPTISOL GS also remained stable with the formation of a halo with a diameter equal to 92% of the initial one for up to seven days of storage at 4°C without agitation. In contrast, after nine days a 44% reduction in the diameter of the inhibition halo was observed on the candida albicans plates. After 14 days and without agitation of the bottles, solution 1 did not form any halo. At the end of the experiment (after fourteen days), agitation of the bottles of solution 1 in OPTISOL-GS allowed the recovery of the antimycotic activity of solution 1 with the appearance of inhibition halos corresponding to approximately 46% of the initial diameter.

Solution 1 in OPTISOL-GS also appeared clear when visually assessed for up to nine days of storage, whilst after fourteen days a yellow precipitate formed.

Solution 2 in OPTISOL-GS remained stable for up to 2 days of storage at 4°C without agitation. After seven, nine and 14 days, approximately a 25%, 55% and 65% reduction in the diameter of the inhibition halos was observed on the candida albicans plates. At the end of the experiment (after fourteen days), agitation of the bottles of solution 2 in OPTISOL-GS allowed the recovery of the antimycotic activity to be highlighted, with the appearance of inhibition halos corresponding to approximately 55% of the initial diameter. Solution 2 in OPTISOL-GS also appeared clear when visually assessed for up to nine days of storage, whilst after fourteen days a yellow precipitate formed.

Below are the results for the solutions in EUSOL-C.

As can be seen, in this case the activity of solution 1 was comparable with the activity of solution 2 at each time analysed. The only difference was observed after the agitation performed at the end of the fourteen days of storage at 4°C. In fact, after agitation, only solution 2 formed inhibition halos with a diameter equal to 51% of the initial diameter, whilst solution 1 did not form any inhibition halo, indicating a possible degradation of the Amphotericin B-Chondroitin in EUSOL-C. The activity of solution 1 in EUSOL-C remained stable for up to 2 days of storage at 4°C without agitation. In contrast, after seven, nine and fourteen days, approximately a 38%, 69% and 100% reduction in the diameter of the halos was observed respectively. Finally, solution 1 in EUSOL-C appeared clear for the entire period of the experiment.

Again in EUSOL-C, the activity of solution 2 remained stable for up to 2 days of storage 4°C without agitation. In contrast, after seven, nine and fourteen days, approximately a 37%, 70% and 94% reduction in the diameter of the halos was observed. Agitation of the bottle of solution 2 in EUSOL-C at the end of the experiment allowed the recovery of approximately 52% of the initial activity. Solution 2 in EUSOL-C remained clear for up to nine days of storage. In contrast, after fourteen days a yellow precipitate formed.

Finally, it should be noticed that solution 4, used as a control solution, formed halos with a diameter equal to 78% of the initial diameter after fourteen days of storage at 4°C without agitation (Figure 4).

In light of the results shown in the figures, it may, however, be concluded that the molecule according to this invention, which was the subject of the tests, remained active at least for up to nine days of storage at 4°C both in OPTISOL and in EUSOL-C, at the same time being more stable in OPTISOL GS than in EUSOL-C.

In fact, whilst in OPTISOL-GS after fourteen days of storage it showed a 55% loss of activity and with the formation of a concentrations gradient (highlighted by the change in results after agitation), in EUSOL-C it showed a gradual loss of activity of 37% (at seven days), 68% (at nine days) and 100% (at fourteen days) combined with a probable degradation of the molecule.

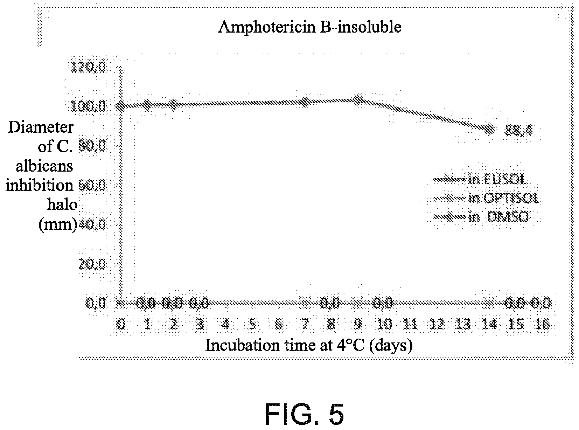

Finally, Figure 5 shows the trend of the inhibition halos for the solutions 3 (both in OPTISOL-C and in EUSOL-C) and for the solution 5.

As can be seen, whilst the solution 5 remained stable for up to nine days of storage at 4°C and after fourteen days an 11% reduction in the diameter of the halos was observed, the solutions 3 did not form any inhibition halo on the candida albicans plates.

The solution 5 also remained clear for the entire period of the experiment. In light of the results indicated above, it may therefore be concluded that the antimycotic molecule according to this invention achieved the results hoped for and therefore brings important advantages.

In fact, the synthetic antimycotic molecule according to this invention is biocompatible with human organs and tissues (at least for the contact times involved in the relevant applications) and has a high level of solubility in water, combined with significant antimycotic action at least at biocompatible temperatures (approximately between 0°C and 40°C).

Second, thanks to this invention it was possible to develop a water-based composition for storing and/or processing corneas and/or other explanted tissues or organs which has excellent antimycotic properties, properties that in contrast are absolutely absent in the compositions currently on the market.

Furthermore, thanks to this invention it was possible to develop an antimycotic medicine that can easily be administered in an aqueous solution. Finally, it should be noticed that this invention is relatively easy to produce and that even the cost linked to implementing the invention is not very high.

## Claims

1. A synthetic antimycotic molecule constituted of a chain of n basic units, wherein the number n of basic units is equal to a number of repeating units of a molecule of chondroitin sulphate, each basic unit having the structure: 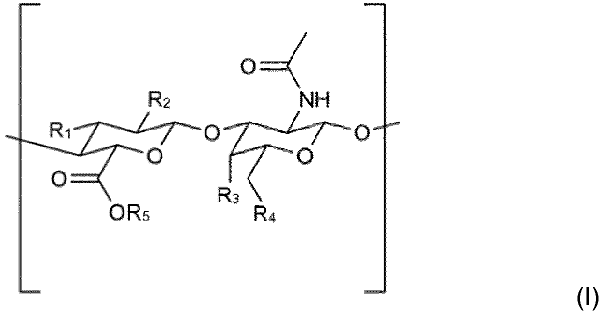 wherein:
- for each basic unit of the chain, one of either R₃ or R₄ is a sulphate group with the structure 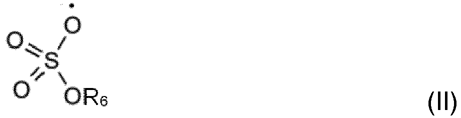 with R₆ representing either a hydrogen atom, or one of either a metallic atom or a NH₄ group, bound to the corresponding oxygen atom O with a monovalent ionic bond;
- for a plurality of basic units of the chain, at least one of R₁, R₂, or the one of either R₃ or R₄ which is not said sulphate group, is a group with antimycotic properties in turn having the structure: 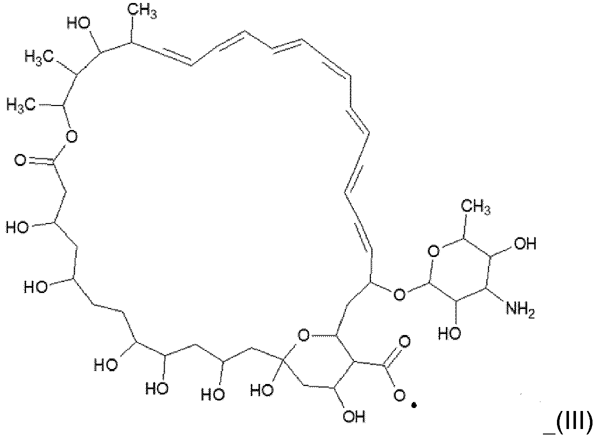
- for each basic unit of the chain, R₅ represents either a hydrogen atom, or one of either a metallic atom or a NH₄ group, bound to the corresponding oxygen atom O with a monovalent ionic bond;
- and at least most of the remaining R₁, R₂, R₃ and R₄ of the entire chain are each constituted of a -OH group.

2. The antimycotic molecule according to claim 1 **characterised in that** each basic unit of the chain comprises a maximum of only one of said groups with antimycotic properties.

3. The antimycotic molecule according to claim 1 **characterised in that** each basic unit of the chain comprises a maximum of two of said groups with antimycotic properties.

4. The antimycotic molecule according to any one of the preceding claims **characterised in that** only between 1% and 50% of the basic units of the chain comprise at least one of said groups with antimycotic properties.

5. The antimycotic molecule according to any one of the preceding claims **characterised in that** for each basic unit of the chain the sulphate group is group R₄.

6. The antimycotic molecule according to any one of the preceding claims **characterised in that** all of said remaining R₁, R₂, R₃ and R₄ are each constituted of a -OH group.

7. The antimycotic molecule according to any one of the preceding claims for use as a medicine.

8. The antimycotic molecule according to any one of the preceding claims for use in the treatment of mycotic infections or for use in ophthalmic surgery.

9. An aqueous mixture comprising an antimycotic molecule according to any one of claims 1 to 6.

10. The aqueous mixture according to claim 9 **characterised in that** it comprises the antimycotic molecule in a weight/volume percentage of between 1% and 30%.

11. The aqueous mixture according to claim 9 or 10 **characterised in that** it is in the form of hydrogel at least within a range of temperatures between 0°C and 40°C, or **in that** the antimycotic molecule is dissolved in the water of the mixture.

12. The aqueous mixture according to any one of claims 9 to 11 for use as a medicine.

13. The aqueous mixture according to any one of claims 9 to 11 for use in the treatment of mycotic infections
**or** for use in ophthalmic surgery.

14. A liquid for storing and/or processing organs or tissues explanted from a first subject, which are intended for transplantation in a second subject who is different from the first subject, comprising a molecule according to any one of claims 1 to 8 or a mixture according to any one of claims 9 to 13.

15. A non-therapeutic method for treating organs or tissues explanted from a first subject, which are intended for transplantation in a second subject who is different from the first subject, wherein the organ or tissue is immersed in a water-based composition comprising an antimycotic molecule according to any one of claims 1 to 8 or a mixture according to any one of claims 9 to 13.

## Patentansprüche

1. Ein synthetisches antimykotisches Molekül, aus einer Kette von n Grundeinheiten bestehend, wobei die Anzahl n der Grundeinheiten gleich einer Anzahl sich wiederholender Einheiten eines Chondroitinsulfat-Moleküls ist, wobei jede Grundeinheit folgende Struktur hat: 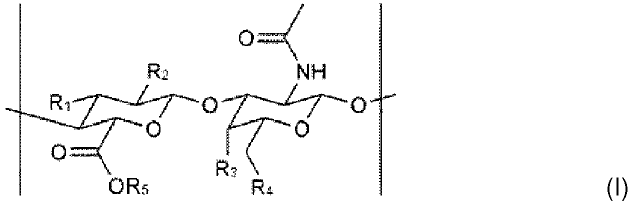 wobei:
- bei jeder Grundeinheit der Kette entweder R₃ oder R₄ eine Sulfatgruppe mit folgender Struktur ist 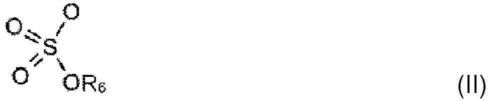 dabei stellt R₆ entweder ein Wasserstoffatom dar, oder entweder ein Metallatom oder alternativ eine NH₄-Gruppe, die mit einer monovalente lonenbindung mit dem entsprechenden Sauerstoffatom O verbunden ist;
- bei einer Mehrzahl von Grundeinheiten der Kette mindestens eine aus R₁, R₂ oder diejenige aus entweder R₃ oder R₄, die nicht besagte Sulfatgruppe ist, eine Gruppe mit antimykotischen Eigenschaften ist, welche ihrerseits folgende Struktur hat: 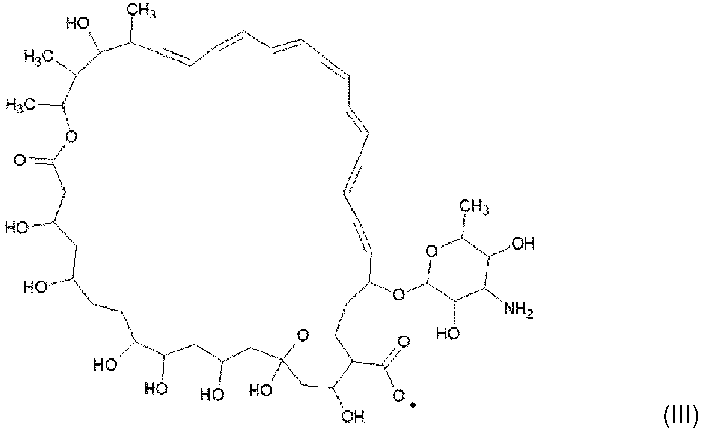
- bei jeder Grundeinheit der Kette stellt R₅ entweder ein Wasserstoffatom oder entweder ein Metallatom oder alternativ eine NH₄-Gruppe dar, die mit einer monovalenten Ionenbindung mit dem entsprechenden Sauerstoffatom O verbunden ist;
- und mindestens die meisten der verbleibenden R₁, R₂, R₃ und R₄ der gesamten Kette bestehen jeweils aus einer -OH-Gruppe.

2. Das antimykotische Molekül nach dem Patentanspruch 1, **gekennzeichnet dadurch, dass** jede Grundeinheit der Kette maximal eine einzige der besagten Gruppen mit antimykotischen Eigenschaft umfasst.

3. Das antimykotische Molekül nach dem Patentanspruch 1, **gekennzeichnet dadurch, dass** jede Grundeinheit der Kette maximal zwei der besagten Gruppen mit antimykotischen Eigenschaften umfasst.

4. Das antimykotische Molekül nach jedem der vorherigen Patentansprüche, **gekennzeichnet dadurch, dass** nur zwischen 1 % und 50 % der Grundeinheiten der Kette mindestens eine der besagten Gruppen mit antimykotischen Eigenschaften umfassen.

5. Das antimykotische Molekül nach jedem der vorherigen Patentansprüche, **gekennzeichnet dadurch, dass** bei jeder Grundeinheit der Kette die Sulfatgruppe die Gruppe R₄ ist.

6. Das antimykotische Molekül nach jedem der vorherigen Patentansprüche, **gekennzeichnet dadurch, dass** bei allen der besagten verbleibenden R₁, R₂, R₃ und R₄ jedes einzelne aus einer -OH-Gruppe besteht.

7. Das antimykotische Molekül nach jedem der vorherigen Patentansprüche zur Verwendung als Medikament.

8. Das antimykotische Molekül nach jedem der vorherigen Patentansprüche zur Verwendung in der Behandlung von Pilzinfektionen oder zur Verwendung in der Augenchirurgie.

9. Eine wässrige Mischung, ein antimykotisches Molekül nach jedem der Patentansprüche 1 bis 6 beinhaltend.

10. Die wässrige Mischung nach dem Patentanspruch 9, **gekennzeichnet dadurch, dass** sie das antimykotische Molekül in einem Gewicht-Volumen-Prozentsatz zwischen 1 % und 30 % beinhaltet.

11. Die wässrige Mischung nach dem Patentanspruch 9 oder 10, **gekennzeichnet dadurch, dass** sie die Form eines Hydrogels hat, zumindest innerhalb eines Temperaturbereichs zwischen 0° C und 40° C, oder dass das antimykotische Molekül im Wasser der Mischung aufgelöst ist.

12. Die wässrige Mischung nach jedem der Patentansprüche 9 bis 11 zur Verwendung als Medikament.

13. Die wässrige Mischung nach jedem der Patentansprüche 9 bis 11 zur Verwendung in der Behandlung von Pilzinfektionen
**oder** zur Verwendung in der Augenchirurgie.

14. Eine Flüssigkeit zur Aufbewahrung und/oder Verarbeitung von Organen oder Geweben, die aus einem ersten Subjekt explantiert wurden, die zur Transplantation in ein zweites Subjekt vorgesehen sind, welches sich vom ersten Subjekt unterscheidet, ein Molekül nach jedem der Patentansprüche 1 bis 8 oder eine Mischung nach jedem der Patentansprüche 9 bis 13 beinhaltend.

15. Ein nicht-therapeutisches Verfahren zur Behandlung von Organen oder Geweben, die aus einem ersten Subjekt explantiert wurden, die zur Transplantation in ein zweites Subjekt vorgesehen sind, welches sich vom ersten Subjekt unterscheidet, wobei das Organ oder das Gewebe in eine Zusammensetzung auf Wassergrundlage eingetaucht ist, welche ein antimykotisches Molekül nach jedem der Patentansprüche 1 bis 8 oder eine Mischung nach jedem der Patentansprüche 9 bis 13 beinhaltet.

## Revendications

1. Une molécule antimycotique synthétique constituée d'une chaîne de n unités de base, dans laquelle le nombre n d'unités de base est égal à un nombre d'unités répétées d'une molécule de sulfate de chondroïtine, chaque unité de base ayant la structure : 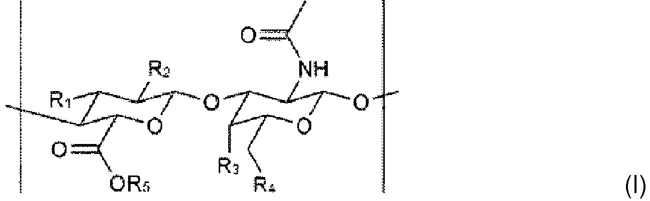 où :
- pour chaque unité de base de la chaîne, l'un ou l'autre de R₃ ou R₄ est un groupe sulfate avec la structure 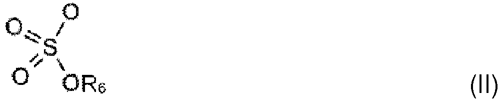 avec R₆ qui représente soit un atome d'hydrogène, soit l'un ou l'autre entre un atome métallique ou un groupe NH₄, lié à l'atome d'oxygène O correspondant avec une liaison ionique monovalente ;
- pour une pluralité d'unités de base de la chaîne, au moins l'un de R₁, R₂, ou celui entre R₃ ou R₄ qui n'est pas ledit groupe sulfate, est un groupe avec des propriétés antimycotiques ayant la structure: 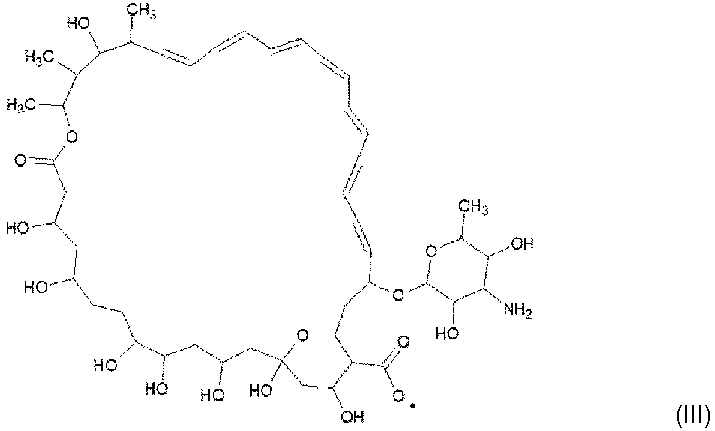
- pour chaque unité de base de la chaîne, R₅ représente soit un atome d'hydrogène, soit l'un ou l'autre entre un atome métallique ou un groupe NH₄, lié à l'atome d'oxygène O correspondant avec une liaison ionique monovalente ;
- et au moins la plupart des R₁, R₂, R₃ et R₄ restants de toute la chaîne sont constitués chacun d'un groupe -OH.

2. La molécule antimycotique selon la revendication 1, **caractérisée en ce que** chaque unité de base de la chaîne comprend un maximum de seulement un desdits groupes avec propriétés antimycotiques.

3. La molécule antimycotique selon la revendication 1, **caractérisée en ce que** chaque unité de base de la chaîne comprend un maximum de deux desdits groupes avec propriétés antimycotiques.

4. La molécule antimycotique selon l'une quelconque des revendications précédentes, **caractérisée en ce que** seulement entre 1 % et 50 % des unités de base de la chaîne comprennent au moins un desdits groupes avec propriétés antimycotiques.

5. La molécule antimycotique selon l'une quelconque des revendications précédentes, **caractérisée en ce que** pour chaque unité de base de la chaîne le groupe sulfate est le groupe R₄.

6. La molécule antimycotique selon l'une quelconque des revendications précédentes, **caractérisée en ce que** tous lesdits R₁, R₂, R₃ et R₄ restants sont constitués chacun d'un groupe -OH.

7. La molécule antimycotique selon l'une quelconque des revendications précédentes destinée à être utilisée en tant que médicament.

8. La molécule antimycotique selon l'une quelconque des revendications précédentes destinée à être utilisée dans le traitement d'infections mycotiques ou à être utilisée en chirurgie ophtalmique.

9. Un mélange aqueux comprenant une molécule antimycotique selon l'une quelconque des revendications de 1 à 6.

10. Le mélange aqueux selon la revendication 9, **caractérisé en ce qu'**il comprend la molécule antimycotique dans un pourcentage poids/volume compris entre 1 % et 30 %.

11. Le mélange aqueux selon la revendication 9 ou 10, **caractérisé en ce qu'**il est sous forme d'hydrogel au moins dans une plage de températures comprises entre 0°C et 40°C, ou **en ce que** la molécule antimycotique est dissoute dans l'eau du mélange.

12. Le mélange aqueux selon l'une quelconque des revendications de 9 à 11 destiné à être utilisé en tant que médicament.

13. Le mélange aqueux selon l'une quelconque des revendications de 9 à 11 destiné à être utilisé dans le traitement d'infections mycotiques ou à être utilisé en chirurgie ophtalmique.

14. Un liquide pour conserver et/ou traiter des organes ou tissus explantés d'un premier sujet, qui sont destinés à être greffés dans un deuxième sujet qui est différent du premier sujet, comprenant une molécule selon l'une quelconque des revendications de 1 à 8 ou un mélange selon l'une quelconque des revendications de 9 à 13.

15. Un procédé non thérapeutique pour traiter des organes ou tissus explantés d'un premier sujet, qui sont destinés à être greffés dans un deuxième sujet qui est différent du premier sujet, dans lequel l'organe ou tissu est immergé dans une composition à base d'eau comprenant une molécule antimycotique selon l'une quelconque des revendications de 1 à 8 ou un mélange selon l'une quelconque des revendications de 9 à 13.
